(19) Europäisches Patentamt
European Patent Office
Office européen des brevets

(11) **EP 3 925 530 A1**

(12) **EUROPEAN PATENT APPLICATION**
published in accordance with Art. 153(4) EPC

(43) Date of publication:
**22.12.2021 Bulletin 2021/51**

(21) Application number: **20756580.5**

(22) Date of filing: **10.02.2020**

(51) Int Cl.:
**A61B 5/05** *(2021.01)*        **A61B 5/107** *(2006.01)*

(86) International application number:
**PCT/JP2020/005109**

(87) International publication number:
**WO 2020/166554 (20.08.2020 Gazette 2020/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.02.2019 JP 2019022352**

(71) Applicant: **Tanita Corporation**
**Tokyo 174-8630 (JP)**

(72) Inventors:
• **WACHI, Yuto**
  **Tokyo 174-8630 (JP)**
• **KASAHARA, Yasuhiro**
  **Tokyo 174-8630 (JP)**

(74) Representative: **Eisenführ Speiser**
**Patentanwälte Rechtsanwälte PartGmbB**
**Postfach 10 60 78**
**28060 Bremen (DE)**

(54) **BODY SHAPE DATA ACQUISITION SYSTEM, BODY SHAPE DATA ACQUISITION PROGRAM, AND COMPUTER-READABLE NON-TRANSITORY STORAGE MEDIUM**

(57)    The present disclosure provides a body shape data acquisition system that can acquire body shape data in a simplified manner. A body shape data acquisition system (50) includes an input unit (51) and a measurement unit (52) that acquire biometric information of a user, a storage unit (54) that stores a plurality of reference body shape data corresponding to the biometric information, a selection unit (53) that selects, from the reference body shape data stored in the storage unit (54), the reference body shape data corresponding to the user's biometric data acquired by the input unit (51) and the measurement unit (52), and an acquisition unit (55) that acquires the user's body shape data using the reference body shape data selected by the selection unit (53).

50

BODY SHAPE DATA ACQUISITION SYSTEM

51 — INPUT UNIT
54 — MEMORY UNIT
56 — MODIFICATION INSTRUCTION RECEPTION UNIT
57 — DISPLAY UNIT
52 — MEASUREMENT UNIT
53 — SELECTION UNIT
55 — ACQUISITION UNIT

Fig.3

EP 3 925 530 A1

**Description**

CROSS-REFERENCE TO RELATED APPLICATIONS

This application claims the benefit of Patent Application No. 2019-022352, filed in Japan on February 12, 2019, the contents of which are hereby incorporated by reference.

TECHNICAL FIELD

The present disclosure relates to a body shape data acquisition system for acquiring body shape data of a user, a body shape data acquisition program, and a computer-readable non-transitory recording medium recording the same.

BACKGROUND TECHNOLOGY

A three-dimensional human body model representing the body shape of an individual is effectively used in various fields such as healthcare, games, and apparel. Body shape data for generating a human model of an individual can be obtained by optically scanning the human body in 3D. In addition, Japanese patent JP5990820B discloses a device for obtaining body shape data by calculating body composition such as lean mass and fat mass of each part such as arms and legs from biometric information (body weight, bioelectrical impedance (BIA), etc.) obtained by measurement, and determining the size of each part and the size of fat of each part, etc. based on these data.

SUMMARY

However, the method of obtaining body shape data by optically scanning the human body in three dimensions requires either marking the human body (a marked suit may be worn) or a special camera such as a stereo camera in order to perform the three dimensional scanning. In addition, the method of JP5990820B requires a measurement device that can perform complex calculations to calculate the size of each part of the body and the size of the fat in each part. In addition, the method of JP5990820B requires more detailed calculations for each part of the body, which is time-consuming when trying to obtain more accurate body shape data.

The method of JP5990820B obtains body shape data by deforming a reference model such as a cylinder or elliptical weight for each part of the body such as arms, legs, etc., based on the body composition such as length, fat content, lean mass, etc. However, the reference model may deviate from the actual body shape, and in such cases, the amount of change to the reference model becomes too large and errors are likely to occur.

The purpose of the present disclosure is to provide a system for acquiring body shape data that enables simple acquisition of body shape data.

A body shape data acquisition system in one aspect comprises: a biometric information acquisition unit configured to acquire biometric information of a user; a storage unit configured to store a plurality of reference body shape data corresponding to biometric information; a selection unit configured to select the reference body shape data corresponding to the biometric information acquired by the biometric information acquisition unit from the reference body shape data stored in the storage unit; and a body shape data acquisition unit configured to acquire the body shape data of the user using the reference body shape data selected by the selection unit.

With this configuration, since a body shape data of a user is obtained by preparing a plurality of reference body shape data corresponding to biometric information and selecting from among them the reference body shape data corresponding to the user's biometric information, the body shape data can be acquired easily without obtaining the size of each part of the user's body. The body shape data acquisition unit may use the selected reference body shape data as is as the user's body shape data, or it may use the selected reference body shape data with some modification or adjustment as the user's body shape data.

In the above body shape data acquisition system, the selection unit may be configured to select a plurality of the reference body shape data corresponding to the biometric data obtained by the biometric information acquisition unit, and the body shape data acquisition unit may be configured to generate composite body shape data by combining the plurality of the reference body shape data selected by the selection unit, and acquire the body shape data using the composite body shape data.

This configuration enables acquisition of body shape data that corresponds more closely to the user's biometric data compared to the case where a single reference body shape data is used as is. The body shape data acquisition unit may use the composite body shape data as it is as the user's body shape data, or may use the composite body shape data with some modification or adjustment as the user's body shape data.

In the above body shape data acquisition system, the selection unit may be configured to select one of the reference body shape data corresponding to the biometric data acquired by the biometric information acquisition unit, and the body shape data acquisition unit may be configured to obtain the body shape data by modifying the one reference body shape data selected by the selection unit.

With this configuration, a user's body shape model can be obtained by modifying the reference body shape data to match the user's body shape. This modification makes it possible to adjust the size of body parts, especially those that tend to have low estimation accuracy due to weak correlation with biometric data. The body shape data acquisition unit may use the modified reference body shape data as it is as the user's body shape data, or it may further modify or adjust the modified reference body shape data as the user's body shape data.

In the above body shape data acquisition system, the body shape data acquisition unit may be configured to acquire the body shape data by modifying the composite body shape data.

With this configuration, a user's body shape model can be obtained by modifying the reference body shape data to match the user's body shape. This modification makes it possible to adjust the size of body parts, especially those that tend to have low estimation accuracy due to weak correlation with biometric data. The body shape data acquisition unit may use the modified composite body shape data as it is as the user's body shape data, or it may further modify or adjust the modified composite body shape data as the user's body shape data.

In the above body shape data acquisition system, the body shape data acquisition unit may be configured to perform the modification according to modification instruction, and the storage unit may be configured to store contents of the modifications.

With this configuration, the content of the modification according to the modification instructions can be stored.

In the above body shape data acquisition system, the body shape data acquisition unit may be configured to perform the modification according to the operation input, information representing the body shape of the user, or the contents of the modification stored in the storage unit as the modification instruction.

With this configuration, instructions for modification can be given by various means. In particular, when the modification is made in accordance with the content of the modification stored in the memory, the modification is made in accordance with the content of the modification made in the past, thus eliminating the need to give instructions for modification each time body shape data is acquired. The information representing the body shape may be the size of the user's clothes.

In the above body shape data acquisition system, the body shape data acquisition unit may be configured to modify size and/or posture of the skeleton.

With this configuration, body shape data closer to the user's body can be acquired by adjusting the size and posture of the skeleton, which are weakly correlated with biometric data.

The above body shape data acquisition system may further comprise a display unit configured to display a human body model based on the body shape data, wherein the body shape data acquisition unit may be configured to perform the modification according to the modification instruction by the operation input, and the display unit may be configured to receive the operation input and display a screen including the human body model reflecting the modification.

With this configuration, the user can perform the operation input for the modification instruction while checking the human body model on the screen that reflects the modification.

In the above body shape data acquisition system, the storage unit may be a database configured to store the biometric information and the reference body shape data in correspondence with each other, and the selection unit may be configured to identify the biometric information having a close Euclidean distance to the biometric information acquired by the biometric information acquisition unit from the biometric information stored in the database, and select the reference body shape data corresponding to the biometric information identified in the database.

With this configuration, the reference body shape data can be selected by identifying the biometric information that is close to the acquired biometric information among the biometric information stored in the database using the Euclidean distance.

In the above body shape data acquisition system, the storage unit may be a database configured to store the biometric information and the reference body shape data in correspondence with each other, the selection unit may be configured to identify the biometric data that is close to the biometric data acquired by the biometric data acquisition unit from among the biometric data stored in the storage unit using a composite vector amount of new eigenvectors with the biometric data acquired by the biometric data acquisition unit as the origin and processed by principal component analysis, select the reference body shape data corresponding to the biometric information identified in the database.

With this configuration, the reference body shape data can be selected by identifying the biometric information that is close to the acquired biometric information among the biometric information stored in the database by applying principal component analysis to the biometric information.

In the above body shape data acquisition system described above, the storage unit may be configured to store a prediction algorithm learned with the biometric information as input and the reference body shape data corresponding to the biometric information as output, and the selection unit may be configured to select the output obtained by inputting the biometric information acquired by the biometric information acquisition unit into the prediction algorithm as the reference body shape data corresponding to the biometric information acquired by the biometric information acquisition unit.

With this configuration, reference body shape data corresponding to the acquired biometric information can be selected using a machine learning model.

In the above body shape data acquisition system, the biometric information may be at least one of the following: gender, age, height, weight, hands-to-feet bioelectrical impedance, both-hands bioelectrical impedance, both-feet bioelectrical impedance, fat mass, lean mass, fat thickness, and muscle mass.

With this configuration, body shape data can be obtained from gender, age, height, weight, or bioelectrical impedance, which are relatively easy to obtain, or from information such as the amount of fat, which is calculated from bioelectrical impedance.

A body shape data acquisition program in one aspect enables a computer equipped with a storage unit configured to store a plurality of reference body shape data corresponding to biometric information to function as: a biometric data acquisition unit configured to acquire biometric data of a user; a selection unit configured to select, from the reference body shape data stored in the storage unit, a reference body shape data corresponding to the biometric information acquired by the biometric information acquisition unit; and a body shape data acquisition unit configured to acquire the body shape data of the user using the reference body shape data selected by the selection unit.

With this configuration, since a body shape data of a user is obtained by preparing a plurality of reference body shape data corresponding to biometric information and selecting from among them the reference body shape data corresponding to the user's biometric information, the body shape data can be acquired easily without obtaining the size of each part of the user's body.

BRIEF DISCRIPTION OF THE DRAWINGS

Fig. 1 shows a body shape data acquisition system of the embodiment;
Fig. 2 shows the usage of the measurement device of the embodiment;
Fig. 3 shows a block diagram of the body shape data acquisition system of the embodiment;
Fig. 4 shows an example of the database of the embodiment;
Fig. 5 shows an example of a human body model based on body shape data of the embodiment;
Fig. 6 shows a calculation of the degree of similarity in the first selection method of the embodiment;
Fig. 7 shows a calculation of the degree of similarity of the embodiment;
Fig. 8 shows a generation of composite body shape data of the embodiment;
Fig. 9 shows a screen for manual modification of the embodiment;
Fig. 10 shows a flowchart of the method for acquiring body shape data of the embodiment; and
Fig. 11 shows a flowchart of the method of acquiring body shape data of the embodiment.

DESCRIPTION OF EMBODIMENTS

The following describes embodiments of the present disclosure with reference to the drawings. The embodiments described below are examples of how the present disclosure may be implemented, and do not limit the present disclosure to the specific configurations described below. In the implementation of the present disclosure, the specific configuration according to an embodiment may be adopted as appropriate.

In this specification, body shape data is data for representing the shape of a body. Although the body shape data may be data that represents the two-dimensional shape of the body, the body shape data in this embodiment is data that represents the three-dimensional shape of the body. When the body shape data is data that expresses a three-dimensional shape, its data format may be wireframe, surface, solid, voxel, or polygon, and its file format may be JSON, STL, PLY, or any other format. Body shape data does not have to be data that expresses a two- or three-dimensional shape by itself, but may be a set of parameters that express the size of each part of the body. In this case, by applying the parameters to a given human body model, a human body model corresponding to the parameters can be obtained.

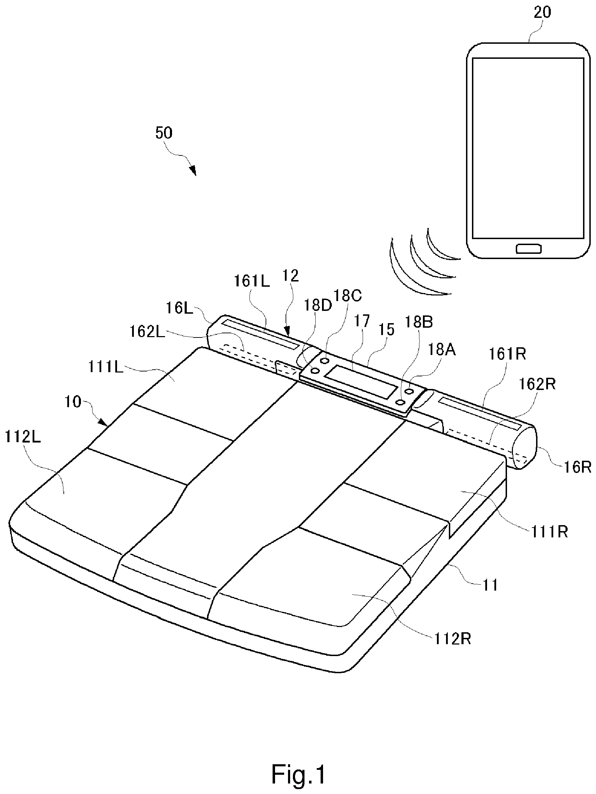

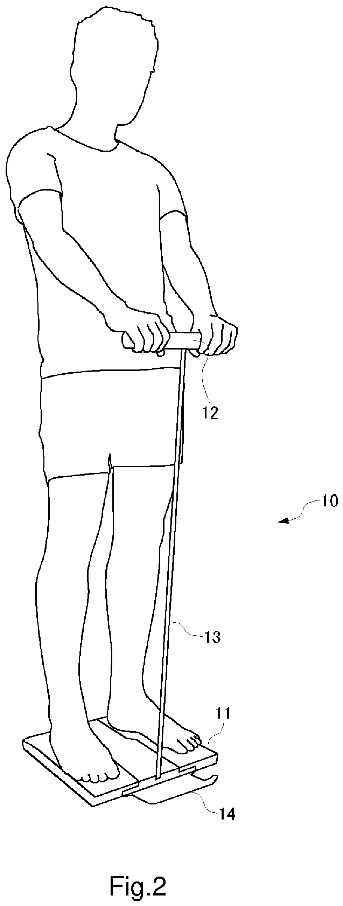

Fig. 1 shows the body shape data acquisition system of the embodiment. Fig. 2 shows usage mode of the measurement device of the embodiment. In this embodiment, a body shape data acquisition system 50 comprises a measurement device 10 and an information processing terminal (hereinafter referred to as a "user terminal") 20. The measurement device 10 is a body composition meter, capable of measuring body weight and body composition as biometric information. The measuring device 10 is equipped with a main unit 11 and a handle unit 12.

The main unit 11 and the handle unit 12 are electrically connected by a connecting line 13. The handle unit 12 can be accommodated in a housing unit 14 provided in the main unit 11. When the handle unit 12 is accommodated in the housing unit 14, the connecting line 13 is wound up by a winding mechanism not shown inside the main unit 11, and is accommodated inside the main unit 11.

The main unit 11 is equipped with a current-carrying electrode 111R and a measuring electrode 112R on the right side of the upper surface, and a current-carrying electrode 111L and a measuring electrode 112L on the left side of the upper surface.

The handle unit 12 has an approximate bar shape, with a handle body 15 in the center of it, and grips 16R and 16L on both sides of the handle body 15. The handle body 15 is provided with a display panel 17 and operation buttons 18A to 18D. The grip 16R is provided with a current-carrying electrode 161R and a measuring electrode 162R, and the grip 16L is provided with a current-carrying electrode 161L and a measuring electrode 162L.

In the usage mode shown in Fig. 2, a user can measure biometric data by standing upright on the main unit 11 with bare feet and grasping the handle unit 12 with both hands with both arms extended in front of him/her. At this time, the base of the right toe comes in contact with the current-carrying electrode 111R, the heel of the right foot comes in contact with the measuring electrode 112R, the base of the left toe comes in contact with the current-carrying electrode 111L, the heel of the right foot comes in contact with the measuring electrode 112L, the palm of the right hand comes in contact with the current-carrying electrode 161R, and the fingers of the right hand come in contact with the measuring electrode 162R, the palm of the left hand comes into contact with the electrode 161L, and the fingers of the left hand come into contact with the electrode 162L.

The main unit 11 is equipped with a load cell inside to measure the weight of the user on the main unit 11 as shown in Fig. 2.

The user terminal 20 is a portable terminal equipped with a computer capable of executing application programs, internal storage such as flash memory, a touch panel, and various connectors. The user terminal 20 is also equipped with a wireless communication device for connecting to the Internet and a short-range communication device for connecting to other nearby devices. The measurement device 10 and the user terminal 20 can send and receive various types of information through short-range wireless communication by pairing with each other.

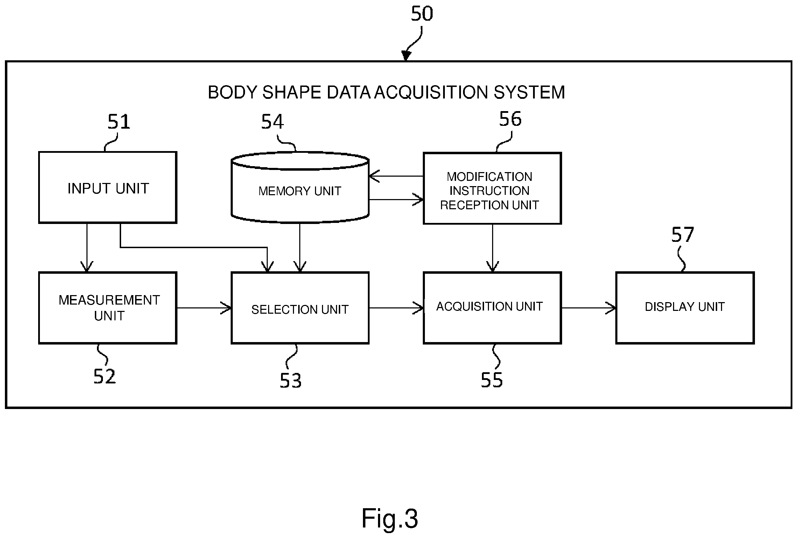

Fig. 3 shows a block diagram of a body shape data acquisition system of the embodiment. The body shape data acquisition system 50 has an input unit 51, a measurement unit 52, a selection unit 53, a memory unit 54, an acquisition unit 55, a modification instruction reception unit 56, and a display unit 57.

The measurement unit 52 is provided in the measurement device 10. As for the configurations other than the measurement unit 52, they may be provided in either the measurement device 10 or the user terminal 20, or all the configurations may be provided in the measurement device 10, and the measurement device 10 alone may constitute the body shape data acquisition system 50. Also, the selection unit 53, the storage unit 54, the acquisition unit 55, and the modification instruction reception unit 56 may be provided in another device that communicates with the measurement device 10 or the user terminal 20 via the Internet, and the body shape data acquisition system 50 may be configured with the other device, the measurement device 10, and the user terminal 20. In this case, the other device may be shared by a plurality of measurement devices 10 or user terminals 20.

In this embodiment, the input unit 51 and the measurement part 52 are provided in the measurement device 10, and the other components are provided in the user terminal 20. In this embodiment, the user terminal 20 is a general-purpose computer, and the selection unit 53, the storage unit 54, the acquisition unit 55, and the modification instruction reception unit 56 are configured in the user terminal 20 by the processor executing an application program running on its operation system. The application program may be provided to the user terminal 20 by the user terminal 20 downloading it from a communication network, or may be provided to the user terminal 20 via a non-transitory recording medium.

The input unit 51 receives operations of turning on and off the power of the measurement device 10, operations of various settings, and operations of inputting biometric information by the user. Specifically, the input unit 51 receives operations of inputting biometric information such as age, gender, height, and the like. The measurement unit 52 measures the user's weight, bioelectrical impedance, and other biometric information. In addition, the measurement unit 52 obtains biometric information such as the fat percentage of the whole body and each body part by applying the biometric information such as the user's height, weight, and bioelectrical impedance to a predetermined regression equation and calculating it. Thus, the biometric information includes that which is input by the user in the input unit 51 (input biometric information), that which is measured in the measurement unit 52 (measured biometric information), and that which is calculated by the measurement unit 52 (calculated biometric information).

The measurement unit 52 is equipped with a load cell for measuring the weight. The load cell is composed of a straining body of a metal member that deforms in response to a load, and a strain gauge that is affixed to the straining body. When a user rides on top of the measurement device 10, the load of the user causes the load cell's diastrophism body to bend and the strain gauge to expand and contract. The resistance value (output value) of the strain gauge changes in accordance with the expansion and contraction. The measurement unit 52 calculates the weight from the difference between the output value of the load cell when no load is applied (zero point) and the output value when a load is applied. The same configuration as that used in general scales can be used for the measurement of the weight using the load cell.

The measurement unit 52 is further provided with each of the above electrodes 111R, 111L, 112R, 112L, 161R, 161L, 162R, 162L, and a current control circuit for applying a current to each of the current-carrying electrodes 161R, 161L, 111R, 111L.

The measurement of the bioelectrical impedance of the whole body and each body part of the user in the measurement unit 52 is performed, for example, in the following manner:

(1) To measure the bioelectrical impedance of the whole body, a current is supplied using the current-carrying electrode 161L and the current-carrying electrode 111L, and in the current path flowing through the left hand, the left arm, the chest, the abdomen, the left leg, and the left foot, the potential difference between the measuring electrode 162L in contact with the left hand and the measuring electrode 112L in contact with the left foot is measured;

(2) To measure the bioelectrical impedance of the right leg, a current is supplied using the current-carrying electrode 161R and the current-carrying electrode 111R, and in the current path flowing through the right hand, the right arm, the chest, the abdomen, the right leg, and right leg, the potential difference between the measuring electrode 112L in contact with the left leg and the measuring electrode 112R in contact with the right leg is measured;

(3) To measure the bioelectrical impedance of the left leg, a current is supplied using the current-carrying electrode 161L and the current-carrying electrode 111L, and in the current path flowing through the left hand, left arm, chest, abdomen, left leg, and left leg, the potential difference between the measuring electrode 112L in contact with the left leg and the measuring electrode 112R in contact with the right leg is measured;

(4) To measure the bioelectrical impedance of the right arm, a current is supplied using the current-carrying electrode 161R and the current-carrying electrode 111R, and in the current path flowing through the right hand, right arm, chest, abdomen, right leg, and right foot, the potential difference between the measuring electrode 162L in contact with the left hand and the measuring electrode 162R in contact with the right hand is measured; and

(5) To measure the bioelectrical impedance of the left arm, a current is supplied using the current-carrying electrode 161L and the current-carrying electrode 111L, and in the current path flowing through the left hand, left arm, chest, abdomen, left leg, and left foot, the potential difference between the measuring electrode 162L in contact with the left hand and the measuring electrode 162R in contact with the right hand is measured.

In this way, the measurement unit 52 passes a current from each current-carrying electrode to a predetermined part of the user's body, measures the potential difference generated in this current path, and calculates the bioelectrical impedance of the user's whole body or each body part based on each of these values of current and potential difference. The configuration for measuring the bioelectrical impedance can be the same as that of a general body composition meter.

In addition, the measurement unit 52 calculates, by applying the input biometric information and the measured biometric information to a predetermined regression equation, the fat percentage, fat mass, lean mass, muscle mass, visceral fat mass, visceral fat level, internal fat area, subcutaneous fat mass, basal metabolic rate, bone mass, body water content, BMI (Body Mass Index), intracellular fluid volume, extracellular fluid volume, etc. Extracellular fluid volume, etc. The same configuration as in general body composition analyzers can be used for the calculation of the calculated biometric data. In addition, the calculated biometric information may be obtained by a machine learning model that inputs the input biometric information and the measured biometric information and outputs the calculated biometric information.

As described above, the input unit 51 acquires biometric data by operation input, and the measurement unit 52 acquires biometric data by measurement or measurement and calculation, both of which function as biometric data acquisition units to acquire biometric data.

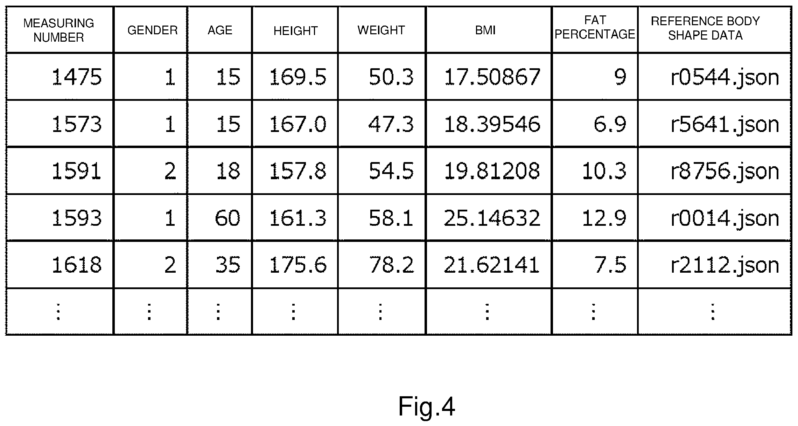

The storage unit 54 is a database in which biometric information and reference body shape data (hereinafter referred to as "reference body shape data") are mapped. Fig. 4 shows an example of the database of the embodiment. In the example of Fig. 4, for each measurement number, one record is composed of the biometric information: gender, age, height, weight, BMI, and fat percentage, which are mapped to the reference body shape data. The biometric information stored in the storage unit 54 may include not only the gender, age, height, weight, BMI, and fat percentage itself, but also their multipliers and ratios.

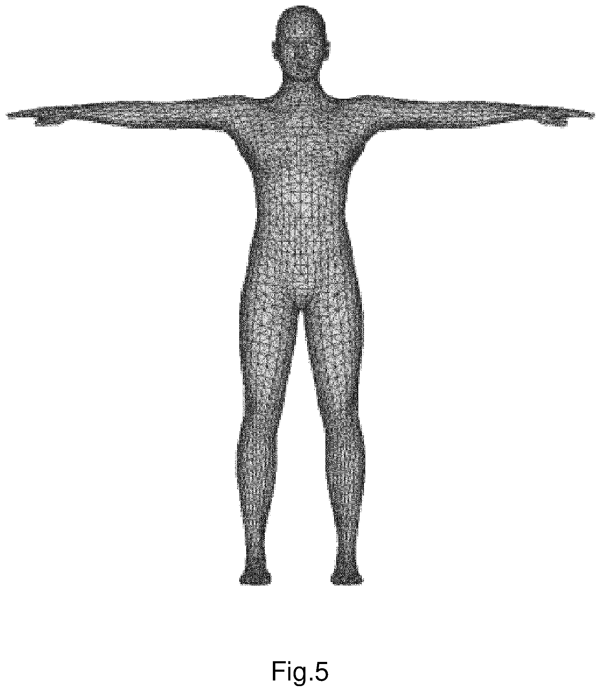

Fig. 5 shows an example of a human body model based on the body shape data of the embodiment. In this example, the body shape data is three-dimensional data in wireframe format.

As shown in Fig. 4, many combinations of biometric data and reference body shape data are stored in the database. In the database, the table in which the measurement number and the biometric information are mapped and the table in which the measurement number and the reference body shape data are mapped may be stored separately.

The selection unit 53 selects, from the reference body shape data stored in the storage unit 54, the reference body shape data corresponding to the biometric information obtained by the input unit 51 and the measurement unit 52. In this embodiment, as described above, since gender, age, height, weight, BMI, and fat percentage are stored in the database of the storage unit 54 in correspondence with the reference body shape data, the selection unit 53 takes out the gender, age, and height of the user entered in the input unit 51, and the weight, BMI, and fat percentage of the user measured in the measurement unit 54, and selects biometric information close to a combination of the information. If the database in the storage unit 54 contains multipliers and ratios for gender, age, height, weight, BMI, and fat percentage, as described above, the selection unit 53 calculates the corresponding multipliers and ratios.

The acquisition unit 55 acquires the user's body shape data using the reference body shape data selected by the selection unit 53. In the following, two methods of selecting the reference body shape data in the selection unit 53 and acquiring the body shape data in the acquisition unit 55 will be described.

(First method of selection of reference body shape data and acquisition of body shape data)

In the first method of selecting reference body shape data and acquiring body shape data, the selection unit 53 selects one reference body shape data, and the acquisition unit 55 acquires body shape data of a user using this reference body shape data. The selection unit 53 searches for biometric information stored in the memory unit 54 (hereinafter also referred to as "stored information") that is similar to the biometric information acquired by the input unit 51 and measurement unit 52 (hereinafter referred to as "acquired data"), and selects the reference body shape data corresponding to the stored information that is most similar to the acquired information. Three examples of this selection method are described below.

(First selection method)

In the first selection method, the selection unit 53 searches for stored information that has a high degree of similarity with the acquired information. The degree of similarity is calculated by the difference between the acquired information and the stored information. Specifically, the degree of similarity is calculated based on the Euclidean distance between the acquired information and the stored information in a multi-dimensional coordinate space with gender, age, height, weight, BMI, and fat percentage as variables.

Equation (1) below is the equation to calculate the similarity in the first selection method.

$$S_i = 1/\sqrt{\left(\sum_j \left(\left|v_{ij} - v_{0j}\right|^2\right)\right)} \qquad \cdots (1)$$

Here, i is the number of the stored information, j is the number of the variable that constitutes the multi-dimensional special coordinate, $S_i$ is the similarity between the i-th stored information and the acquired information, $v_{ij}$ is the vector of the j-th variable of the i-th stored information, and $v_{0j}$ is the vector of the j-th variable of the acquired information. According to this equation (1), the closer the Euclidean distance between the acquired information and the stored information, the greater the similarity Si.

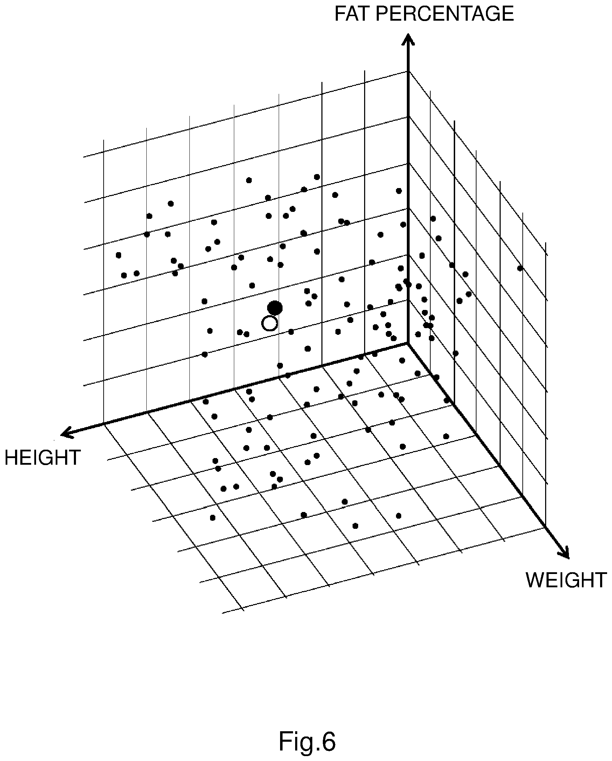

Fig. 6 illustrates the calculation of the similarity in the first selection method of the embodiment. For ease of understanding, the example in Fig. 6 shows a three-dimensional coordinate space with only height, weight, and fat percentage as variables, but if gender, age, height, weight, BMI, and fat percentage are used as variables, a Euclidean distance in a six-dimensional coordinate space will be calculated. In the example in Fig. 6, the hollow circles represent the acquired information, the small dots represent the stored information, and the solid circles represent the stored information that is closest (similar) to the acquired information.

When calculating the similarity by Euclidean distance, weighting may be applied to the difference of variables as shown in Equation (2) below.

$$S_i = 1/\sqrt{\sum_j \left(\left|v_{ij} - v_{0j}\right|^2 /W_j\right)} \qquad \cdots (2)$$

Here, $W_j$ is the weight (importance) of the j-th variable.

The selection unit 53 selects the reference body shape data corresponding to the stored information closest to the acquired information, that is, the stored information with the highest degree of similarity.

(Second selection method)

In the second selection method, the selection unit 53 also searches for the stored information having a high degree of similarity with the acquired information. In the second search method, the acquired information and the stored information are processed by principal component analysis, and the similarity is calculated from the composite vector amount of the new eigenvector with the acquired information as the origin.

Equation (3) below is the equation for calculating the similarity in the second selection method.

$$S_i = 1/\sqrt{\sum_{j^{(pca)}} \left| \mathbf{v}_{ij^{(pca)}} \right|^2} \qquad \cdots (3)$$

Here, $j^{(pca)}$ is the number of the principal component axis that constitutes the multi-dimensional special coordinate, and $v_{ij}^{(pca)}$ is the eigenvector of the i-th stored information for the acquired information.

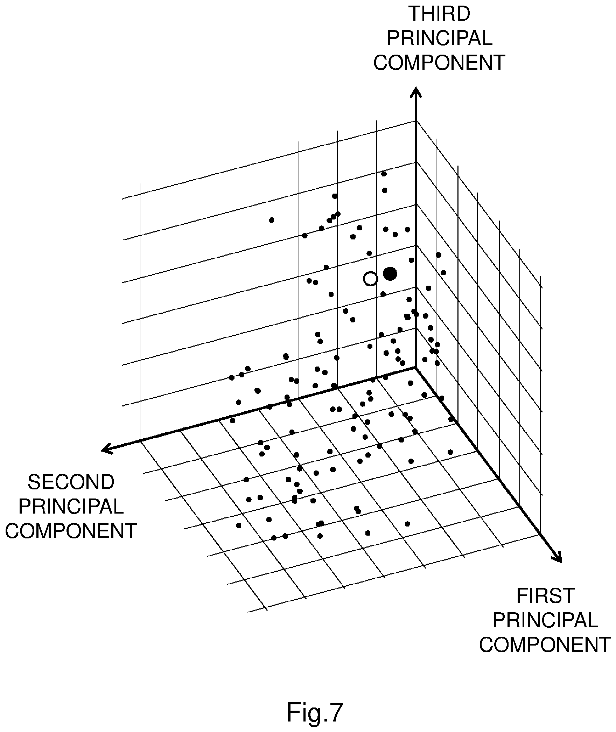

Fig. 7 illustrates the calculation of the similarity of the embodiment. For ease of understanding, the example in Fig. 7 shows a case where principal component analysis is performed on the first through third principal components as variables, but the number of principal components can be larger or smaller than three. In the example in Fig. 7, the hollow circles represent the acquired information, the small dots represent the stored information, and the solid circles represent the stored information that is closest (similar) to the acquired information.

As shown in Equation (4) below, the axes of multiple principal component analysis may be weighted and combined.

$$S_i = 1/\sqrt{\sum_{j^{(pca)}} \left( \left| \mathbf{v}_{ij^{(pca)}} \right|^2 / \mathbf{W}_{j^{(pca)}} \right)} \qquad \cdots (4)$$

Here, $W_j^{(pca)}$ is the weight (importance) of the j-th $^{(pca)}$ principal component axis.

The selection unit 53 selects the reference body shape data corresponding to the stored information closest to the acquired information, i.e., the stored information with the highest degree of similarity. In this embodiment, the similarity was evaluated after processing the stored information and the acquired information by principal component analysis. However, the processing method of the information is not limited to principal component analysis, but can be any processing method by dimension reduction of high dimensional biological information, for example, processing by singular value decomposition.

(Third selection method)

In the third selection method, the storage unit 54 stores a prediction algorithm that has been learned using biometric data as input and reference body shape data corresponding to the biometric data as output. As this prediction algorithm, a prediction algorithm learned by a hierarchical neural network with at least three or more layers, which is constructed with the biometric information as input and the reference body shape data or its features as output, can be employed. In addition to the neural network, other machine learning models such as k-means, SVM (Support Vector Machine), Random Forest, etc. can be employed as the prediction algorithm.

The selection unit 53 selects the output when the acquired information is input to the prediction algorithm as the reference body shape data corresponding to the acquired information.

The acquisition unit 55 acquires the body shape data of the user using the reference body shape data selected by the selection unit 53. The acquisition unit 55 first uses the reference body shape data as is as the user's body shape data. The acquisition unit 55 can also modify this reference body shape data as the user's body shape data. The modification will be described later.

(Second method of selection of reference body shape data and acquisition of body shape data)

In the second method of selecting reference body shape data and acquiring body shape data, the selecting unit 53 selects a plurality of reference body shape data, and the acquisition unit 55 acquires body shape data of a user using the plurality of selected reference body shape data. Specifically, the selection unit 53 searches for a certain number of stored information closest to the acquired information by any of the first to third selection methods described above, and selects a plurality of reference body shape data corresponding to those stored information. The acquisition unit 55 generates the composite body shape data by performing a synthesis process on the plurality of reference body shape data selected by the selection unit 53.

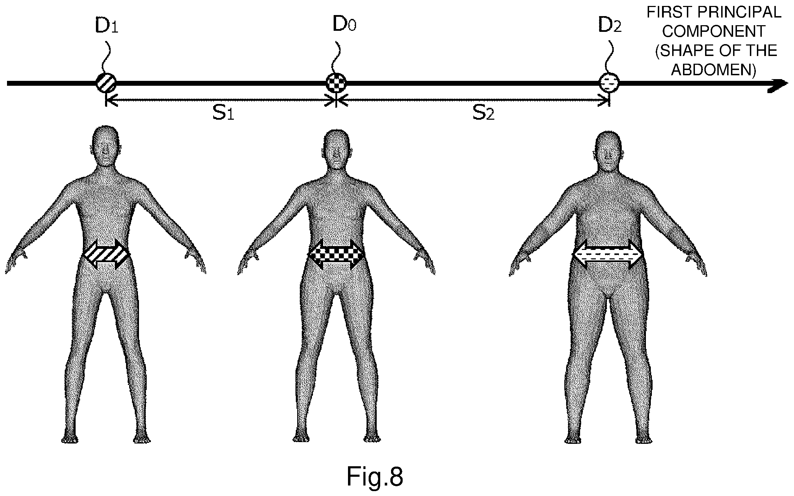

Fig. 8 illustrates the generation of the composite body shape data of the embodiment. For the sake of clarity of explanation, the example in Fig. 8 shows the synthesis process considering only the first principal component axis, which represents the shape of the abdomen. In the example of Fig. 8, the selection unit 53 selects the stored information $D_1$ closest to the acquired information $D_0$ for the first principal component axis and the stored information $D_2$ closest to the acquired information $D_0$ in the opposite direction on the axis from the the $D_1$.

As shown in Fig. 8, in the case where stored information $D_1$ and $D_2$ are obtained as the two stored information closest to the acquired information $D_0$, and their respective similarities are $S_1$ and $S_2$, as shown in Fig. 8, the acquisition unit 55 calculates the composite body shape data $M_c$ as the shape that divides $S_1:S_2$ between the reference body shape data $M_1$ corresponding to the stored information $D_1$ and the reference body shape data $M_2$ corresponding to the stored information $D_2$.

The following is a detailed description of the synthesis process in which the acquisition unit 55 generates the composite body shape data. The acquisition unit 55 calculates the position information of each point of the point group that constitutes the body shape model indicated by the composite body shape data. Specifically, the acquisition unit 55 calculates the positional information of each of the above points, including similarity as a variable for each axis (variable) in a multi-dimensional coordinate space consisting of a plurality of axes (variables). The axes (variables) may be principal component axes. The formula for calculating the position information $P_{kj}$, which includes the similarity of the k-th point k of the point group that constitutes the body shape model indicated by the composite body shape data, in the j-th axis j in the multi-dimensional coordinate space consisting of multiple axes as a variable, is as shown in Equation (5) below.

$$P_{kj} = P_{0,k}^{(1,2,...t)} \times G(S_{c,j}^{(1)}, S_{c,j}^{(2)}, ..., S_{c,j}^{(t)}) \times \alpha_{kj} \qquad \cdots (5)$$

Here, $S_{c,j}^{(1)}$, $S_{c,j}^{(2)}$,..., $S_{c,j}^{(t)}$ are the similarities in axis j between each of the t stored information selected by the selection unit 53 to calculate the similarity in axis j and the acquired information. G is a function whose variable is the degree of similarity, and $\alpha_{kj}$ is the interpolation rate of point k on axis j. In addition, $P_{0,k}^{(1,2,...,t)}$ is the positional information of the points corresponding to point k of the body model indicated by the composite body model among the group of points constituting the body model indicated by the reference body model data corresponding to any one of the t stored information selected to calculate the similarity in axis j. Alternatively, $P_{0,k}^{(1,2,...,t)}$ may be the positional information of the average of each point corresponding to point k of the body model indicated by the composite body model data among the point group constituting each body model indicated by the reference body model data corresponding to a plurality of stored information among the t stored information.

The acquisition unit 55 generates composite body shape data by performing the synthesis process shown in Fig. 8 for each axis (variable) and combining them in a multi-dimensional coordinate space consisting of a plurality of axes (variables). The calculation formula for the positional information Pk, which is a composite of positional information with the similarity on each axis in a multi-dimensional coordinate space consisting of multiple axes of the k-th point k of the point group that constitutes the body model indicated by the composite body shape data as a variable, is shown in Equation (6) below.

$$P_k = \sum_{j=1}^{n} P_{kj} \times C_j \qquad \cdots (6)$$

Here, $C_j$ is the contribution ratio of the j-th axis (variable) used to calculate the similarity to the generation of the composite body shape data.

Regardless of the position of the acquired information, the acquisition unit 55 may perform the synthesis process so that the middle point of the two selected stored information is used as the composite body shape data.

In the case where the reference body shape data is defined by a set of parameters representing the size of each part of the body rather than 3D shape data as described above, the composite body shape data may be generated by interpolating those parameters instead of the synthesis process described above. For example, in the case where the first principal component of the principal component analysis of the stored information represents the size of the waist, if the waists of the two nearest reference body shape data are 63 cm and 65 cm, the waist of the composite body shape data located in the middle of those two reference body shape data may be set to 64 cm.

As described above, in the first method, the acquisition unit 55 acquires one reference body shape data selected by the selecting unit 53, and in the second method, the acquisition unit 55 acquires composite body shape data by synthesizing a plurality of reference body shape data selected by the selecting unit 53. The acquisition unit 55 then has a function to modify these reference body shape data and composite body shape data. The following is a description of the modification method by this modification function.

The modification instruction reception unit 56 receives modification instructions by manual operation of the user on the screen for modification displayed on the display unit 57. The acquisition unit 55 modifies the reference body shape data and composite body shape data according to the manual operation received by the modification instruction reception unit 56. For this modification, the display unit 57 displays a screen for modification that includes a human body model represented by the reference body shape data and the composite body shape data, and a table of sizes of each part that can be modified.

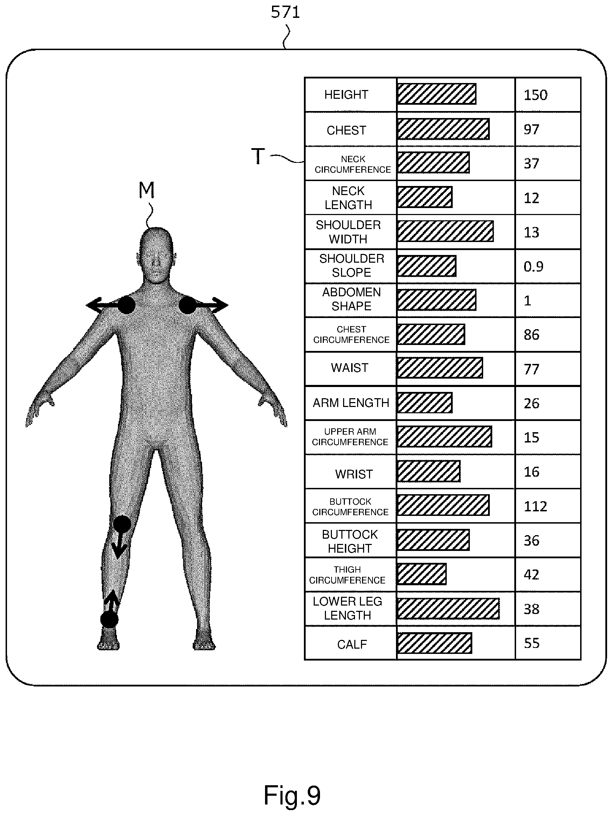

Fig. 9 shows a screen for modification for manual operation of the system. The screen for modification 571 includes a table T of the sizes of each part that can be modified along with the human body model M. The table T shows the current size of each modifiable part as an indicator (bar graph) and a numerical value. In the example of Fig. 9, the sizes that can be modified are mainly those that are difficult to estimate from bioelectrical impedance, such as skeletal sizes such as height, head length, shoulder width, and arm length, as well as neck circumference, wrist, and buttock height. In addition to this, posture such as the bend of the back (hunchback) may also be modifiable.

The user can directly specify each part of the human body model M in this screen for modification 571 to change the size of the corresponding part. For example, as shown in Fig. 9, when a touch operation is performed to pinch out both shoulders of the human body model M, the modification instruction reception unit 56 receives the modification instruction by this operation, and the acquisition unit 55 modifies the reference body shape data and the composite body shape data to increase the shoulder width according to this modification instruction. The display unit 57 displays the human body model M with the shoulder width widened according to the modified reference body shape data and composite body shape data.

As shown in Fig. 9, when the user performs a touch operation to pinch in the lower leg of the human body model M, the modification instruction reception unit 56 receives the modification instruction by this operation, and the acquisition unit 55 modifies the reference body shape data and the composite body shape data so as to shorten the lower leg length in accordance with this modification instruction. The display unit 57 displays the human body model M whose lower leg length has been shortened in accordance with the modified reference body shape data and composite body shape data. If the size of the corresponding part is changed by direct touch operation on the human body model M displayed on the screen 571 for modification, the change is immediately reflected in the table T as well.

In addition, when the indicator is expanded or contracted by dragging the tip (right end) of the indicator in Table T, the display unit 57 will expand or contract the indicator. The modification instruction reception unit 56 receives the modification instruction by this operation, and the acquisition unit 55 modifies the reference body shape data and the composite body shape data so as to modify the corresponding part according to this modification instruction. Accordingly, together with this, the expansion and contraction of the indicator is reflected in the numerical values in Table T. Furthermore, the display unit 57 also reflects the modification of the reference body shape data and composite body shape data in the human body model M.

When an operation is performed to rewrite the numerical values in the table T, the display unit 57 rewrites the numerical values in the table T when the modification instruction reception unit 56 receives the modification instruction from this operation. In response to this, the acquisition unit 55 modifies the reference body shape data and the composite body shape data so as to modify the corresponding part according to this modification instruction. The display unit 57 further expands and contracts the indicator in accordance with the rewritten numerical values, and displays the human body model M reflecting the modification of the reference body shape data and the composite body shape data.

In this way, the modification function of the acquisition unit 55 allows the user to modify the reference body shape data and composite body shape data to match his or her own body shape. In addition, in the screen for modification 571, the user can change the size of each part of the human body by directly manipulating the human body model generated using the reference body shape data and composite body shape data, and can also change the size of each part by stretching and shrinking the indicator for each part, and can also change the size of each part by rewriting the numerical value for each part. In any case, the modified human body model can be checked on the screen for modification 571.

As a variant of the example in Fig. 9, the modification may be instructed by specifying information about the user's body shape, for example, the size of each part of the body such as neck circumference, chest circumference, waist circumference, arm circumference, thigh circumference, etc., or the size of the clothes the user is wearing or body shape type. For example, in the Japanese system, for a business suit, the size can be specified from size 1 to size 10, and the body type can be specified as YA, A, AB, BE, E, or K. In the U.K. and U.S. systems, chest and waist sizes can be specified in the range of 36 to 42, and height categories can be specified as Short (SHT), Regular (REG), and Long (LONG) . In the European system, the chest size can be specified in the range of 42 to 56, the waist size can be specified in the range of Frop8 to Drop0, and the height category can be specified in Corco (C), Regolate (R), and Lungo (L) . For example, in the Japanese system, when the user inputs the size and body shape type to the modification instruction reception unit 56, the acquisition unit 55 modifies the values of shoulder width, waist, etc. according to the size and body shape type.

When the modification instruction reception unit 56 receives the modification instruction and the acquisition unit 55 modifies the reference body shape data and the composite body shape data accordingly, the contents of the modification are stored in the memory unit 54. The contents of the modification stored in the storage unit 54 are referred to the next time the body shape data is acquired according to the same user's acquisition information. That is, if the content of past modifications for the same user is stored in the memory 54, the modification instruction reception unit 56 modifies the reference body shape data and the composite body shape data according to the contents of the modification. In this case, too, the user can further make modifications by manual operation using the screen for modification.

When the reference body shape data or the composite body shape data is modified, a record in which the modified reference body shape data or the composite body shape data is the new reference body shape data together with the biometric information of the user may be stored in the database of the storage unit 54, and the database may be updated in this manner. In particular, as described above, when the storage 54 is provided in another device different from the measurement device 10 and the user terminal 20 and is shared by multiple users, the modifications made by the multiple users are reflected in the database, and the database is effectively updated.

When one reference body shape data is selected by the selection unit 53, the acquisition unit 55 uses the reference body shape data, when a plurality of reference body shape data is selected by the selection unit 53, the acquisition unit 55 generates a composite body shape data by performing a composite processing of the plurality of reference body shape data, and then uses the composite body shape data, when a modification instruction is received by the modification instruction reception unit 56, the reference body shape data and the composite body shape data are further modified, to obtain the user's body shape data.

The display unit 57 displays the human body model using the body shape data acquired by the acquisition unit 55. In this embodiment, the reference body shape data (and the composite body shape data synthesized from the reference body shape data) is data expressed in a wire frame, and the human body model displayed by the display unit 57 is a surface model obtained by converting the wire frame. The data format of the displayed human body model and that of the reference body shape data may be the same, and the display unit 57 may display the reference body shape data, the composite body shape data, or modified data thereof as it is as a human body model. The body shape data acquisition system 50 may not have a display unit 57, but may only transmit the body shape data acquired by the acquisition unit 55 to other devices.

The human body model, when displayed on the display unit 57, can be manipulated for rotation, enlargement and reduction, etc. In addition, the hands and feet of the human body model can be made movable by adding joint information to the body shape data.

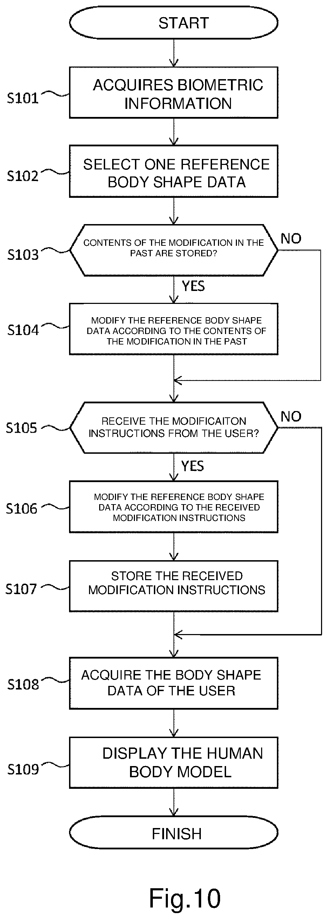

Fig. 10 is a flowchart of the method for acquiring body shape data. The flowchart in Fig. 10 shows an example of the selection unit 53 selecting one reference body shape data. First, the input unit 51 receives operational input of input biometric data such as the user's gender, age, height, etc., and the measurement unit 52 obtains and outputs various biometric data (acquired information) to the selection unit 53 by measuring biometric data such as weight and bioelectrical impedance, and calculating biometric data such as fat percentage (Step S101). Next, the selection unit 53 refers to the database of the storage unit 54, identifies one stored information closest to the acquired information, selects one reference body shape data corresponding to the identified stored information, and outputs it to the acquisition unit 55 (Step S102).

The modification instruction reception unit 56 determines whether or not the contents of the modification made in the past are stored in the memory unit 54 (Step S103), and if they are stored (YES in Step S103), the acquisition unit 55 modifies the reference body shape data with the contents of the modification as the modification instructions (Step S104). If the contents of the modification are not stored in the memory 54 (NO in step S103), and after the modification is made according to the stored contents of the modification, the modification instruction reception unit 56 determines whether or not the modification instructions from the user have been received (step S105).

If a modification instruction from the user is received at the modification instruction reception unit 56 (YES at step S105), the reference body shape data is modified according to the modification instruction (step S106), and the contents of the modification are stored in the memory unit 54 (step S107). If no modification instruction from the user is received (NO at step S105), and after modifying the reference body shape data, the acquisition unit 55 acquires the reference body shape data (or the modified reference body shape data if the modification has been made) as the user's body shape data and outputs it to the display unit 57 (step S108). The display unit 57 displays the human body model according to the body shape data (Step S109).

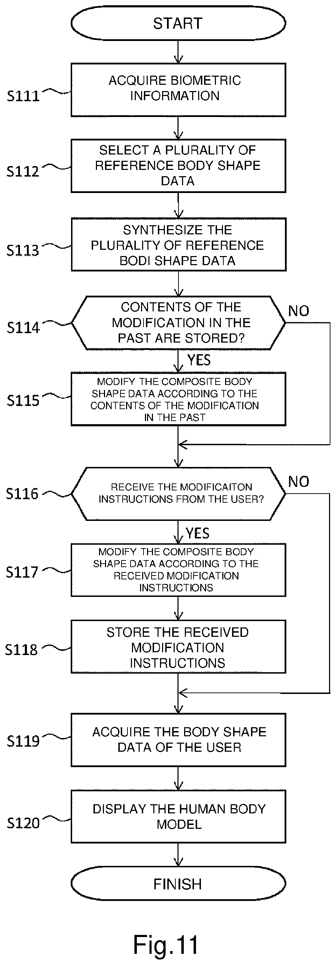

Fig. 11 is a flowchart of a method of acquiring body shape data. The flowchart of Fig. 11 shows an example in which the selection unit 53 selects a plurality of reference body shape data. First, the input unit 51 receives operational input of input biometric data such as the user's gender, age, height, etc., and the measurement unit 52 obtains various biometric data (acquired information) by measuring measured biometric data such as body weight, bioelectrical impedance, etc., and calculating calculated biometric data such as fat percentage, etc., and outputs them to the selection unit 53 (Step S1 11). Next, the selection unit 53 identifies a plurality (e.g., three) of biometric information (stored information) closest to the acquired information by referring to a database in the storage unit 54, and acquires a plurality of reference body shape data corresponding to each of the identified stored information and outputs them to the acquisition unit 55 (Step S112).

The acquisition unit 55 synthesizes the plurality of reference body shape data selected by the selecting unit 53 to generate the composite body shape data (Step S113). The modification instruction reception unit 56 determines whether or not the contents of modifications made in the past are stored in the memory unit 54 (Step S114), and if they are stored (YES in Step S113), the acquisition unit 55 modifies the composite body shape data using the modification contents as the modification instructions (Step S115). If the contents of the modification are not stored in the memory 54 (NO in step S114), and after the modification is made according to the stored modification contents, the modification instruction reception unit 56 determines whether or not the modification instructions from the user have been received (step S116).

If a modification instruction from the user is received at the modification instruction reception unit 56 (YES at step S116), the composite body shape data is modified according to the modification instruction (step S117), and the contents of the modification are stored in the memory unit 54 (step S118). In the case where no modification instruction from the user is received (NO at step S116) and after modifying the composite body shape data, the acquisition unit 55 obtains the user's body shape data according to the composite body shape data (or the modified composite body shape data if modifications have been made) and outputs it to the display unit 57 (Step S119). The display unit 57 displays the human body model according to the body shape data (Step S120).

As described above, according to the body shape data acquisition system 50 of this embodiment, a plurality of reference body shape data are prepared in advance, and when the biometric data of a user is acquired, the body shape data of the user is acquired by selecting reference body shape data corresponding to the biometric data. Therefore, it is sufficient to obtain the user's biometric data to the extent necessary to select the pre-prepared reference body shape data, and thus the user's body shape data can be easily obtained. In the above embodiment, each of the plurality of reference body shape data in the database is associated with gender, age, height, weight, BMI, and fat percentage as biometric information, so it is sufficient to acquire these information as the user's biometric information.

In addition, by reducing the number of records to be stored in the database of the storage unit 54, it is possible to install the storage unit 54 in a small user terminal 20 that has a limited storing capacity.

Even when less information than the biometric data stored in the database is available for a user, e.g., when only gender, age, height, and weight are available as the user's biometric data, it is still possible to search the database for biometric data that is close to such user's biometric data, and it is possible to select the reference body shape data corresponding to the user's biometric data. In addition, in the body shape data acquisition system 50, the measurement unit 52 is not essential, and the user may use his/her own biometric information entered in the input unit 51 and/or biometric information measured by other body composition analyzers to process the above-mentioned selection in the selection unit 53.

Further, the selection unit 53 may identify the attributes of the user, such as height and occupation, from his or her own biometric information input by the user into the input unit 51, and select the stored information corresponding to the biometric information belonging to the same attributes as the user. In this way, since the reference body shape data corresponding to the biometric data belonging to the same attribute as the user is selected, it can be expected that the stored information close to the actual body shape of the user is selected.

When measuring bioelectrical impedance as the user's biometric information, the bioelectrical impedance between hands and feet (whole body) may be measured as in the above embodiment, or only the bioelectrical impedance between both hands may be measured by a device corresponding to the handle unit 12, or only the bioelectrical impedance between both feet may be measured by a device corresponding to the main unit 11. In other words, according to the body shape data acquisition system 50 of this embodiment, even when only the bioelectrical impedance between both hands or between both feet can be obtained and the bioelectrical impedance of the whole body cannot be obtained, the body shape data of the user can be acquired using such bioelectrical impedance.

According to the above embodiment, the human body model of the user can be understood at a glance only by the biometric data of the user obtained by the input unit 51 and the measurement unit 52. If the past human body model and the target human body model are superimposed in the display of the display unit 57, the changes from the past and the difference to the target body shape can be visually understood.

In addition, by outputting body shape data in formats such as ".json", ".blender", ".stl", ".ply", etc., it can be used as character asset data for a game, VR, AR, MR, and other content. Furthermore, by adding various physical characteristics to the body shape data, it is possible to simulate the range of motion of each part of the user and their motor abilities. For example, by adding bones to the body shape data and outputting it as 3D image data in JSON format, it is possible to generate a character with body shape data that represents the user's characteristics in a game application development environment such as unity (registered trademark). Furthermore, by using body composition data such as muscle mass, physical abilities such as jumping power, strength, and stamina can be assigned to the character according to the user's body composition. In the display unit 57, the movements of the character and the human body model may be represented according to the physical abilities.

Furthermore, by storing the history of the user's body shape data in the memory unit 54, the transition of the body shape data can be used to predict the future body shape. In addition, body shape data of athletes, models, etc. can be set as the target body shape, and the degree of similarity and the lineage of similarity between the user's body shape and the target body shape can be known.

EXPLANATIONS OF REFERENCES

| | |
|---|---|
| 10 | Measurement device |
| 11 | Main part |
| 111R, 111L | Current-carrying electrodes |
| 112R, 112L | Measuring electrodes |
| 12 | Handle unit |
| 13 | Connecting line |
| 14 | Housing unit |
| 15 | Handle body |
| 16R, 16L | Grips |
| 161R, 161L | Current-carrying electrodes |
| 162R, 162L | Measuring electrodes |
| 17 | Display panel |
| 18A to 18D | Operation buttons |
| 20 | Information processing terminal (user terminal) |
| 50 | Body shape data acquisition system |
| 51 | Input unit |
| 52 | Measurement unit |
| 53 | Selection unit |
| 54 | Memory unit |
| 55 | Acquisition unit |
| 56 | Modification instruction reception unit |
| 57 | Display unit |
| 571 | Screen for modification |

**Claims**

1. A body shape data acquisition system, comprising:

   a biometric information acquisition unit configured to acquire biometric information of a user;
   a storage unit configured to store a plurality of reference body shape data corresponding to biometric information;
   a selection unit configured to select the reference body shape data corresponding to the biometric information acquired by the biometric information acquisition unit from the reference body shape data stored in the storage unit; and
   a body shape data acquisition unit configured to acquire the body shape data of the user using the reference body shape data selected by the selection unit.

2. The body shape data acquisition system according to claim 1, wherein the selection unit is configured to select a plurality of the reference body shape data corresponding to the biometric data obtained by the biometric information acquisition unit, and
   the body shape data acquisition unit is configured to generate composite body shape data by combining the plurality of the reference body shape data selected by the selection unit, and acquire the body shape data using the composite body shape data.

3. The body shape data acquisition system according to claim 1, wherein the selection unit is configured to select one of the reference body shape data corresponding to the biometric data acquired by the biometric information acquisition unit, and
   the body shape data acquisition unit is configured to obtain the body shape data by modifying the one reference body shape data selected by the selection unit.

4. The body shape data acquisition system according to claim 2, wherein the body shape data acquisition unit is configured to acquire the body shape data by modifying the composite body shape data.

5. The body shape data acquisition system according to claim 3, wherein the body shape data acquisition unit is configured to perform the modification according to modification instruction, and
the storage unit is configured to store contents of the modifications.

6. The body shape data acquisition system according to claim 5, wherein the body shape data acquisition unit is configured to perform the modification according to the operation input, information representing the body shape of the user, or the contents of the modification stored in the storage unit as the modification instruction.

7. The body shape data acquisition system according to any of claims 3 to 6, wherein the body shape data acquisition unit is configured to modify size and/or posture of the skeleton.

8. The body shape data acquisition system according to claim 5, further comprising a display unit configured to display a human body model based on the body shape data, wherein

the body shape data acquisition unit is configured to perform the modification according to the modification instruction by the operation input, and
the display unit is configured to receive the operation input and display a screen including the human body model reflecting the modification.

9. The body shape data acquisition system as described in claim 1, wherein the storage unit is a database configured to store the biometric information and the reference body shape data in correspondence with each other, and
the selection unit is configured to identify the biometric information having a close Euclidean distance to the biometric information acquired by the biometric information acquisition unit from the biometric information stored in the database, and select the reference body shape data corresponding to the biometric information identified in the database.

10. The body shape data acquisition system according to claim 1, wherein the storage unit is a database configured to store the biometric information and the reference body shape data in correspondence with each other,
the selection unit is configured to identify the biometric data that is close to the biometric data acquired by the biometric data acquisition unit from among the biometric data stored in the storage unit using a composite vector amount of new eigenvectors with the biometric data acquired by the biometric data acquisition unit as the origin and processed by principal component analysis, select the reference body shape data corresponding to the biometric information identified in the database.

11. The body shape data acquisition system according to claim 1, wherein the storage unit is configured to store a prediction algorithm learned with the biometric information as input and the reference body shape data corresponding to the biometric information as output, and
the selection unit is configured to select the output obtained by inputting the biometric information acquired by the biometric information acquisition unit into the prediction algorithm as the reference body shape data corresponding to the biometric information acquired by the biometric information acquisition unit.

12. The body shape data acquisition system according to claim 1, wherein the biometric information is at least one of the following: gender, age, height, weight, hands-to-feet bioelectrical impedance, both-hands bioelectrical impedance, both-feet bioelectrical impedance, fat mass, lean mass, fat thickness, and muscle mass.

13. A body shape data acquisition program that enable a computer equipped with a storage unit configured to store a plurality of reference body shape data corresponding to biometric information to function as:

a biometric data acquisition unit configured to acquire biometric data of a user;
a selection unit configured to select, from the reference body shape data stored in the storage unit, a reference body shape data corresponding to the biometric information acquired by the biometric information acquisition unit; and
a body shape data acquisition unit configured to acquire the body shape data of the user using the reference body shape data selected by the selection unit.

A program for acquiring body shape data, which enables the program to function as a body shape data acquisition program.

14. A computer-readable non-transitory storage medium storing a body shape data acquiring program according to claim 13.

Fig.1

Fig.2

EP 3 925 530 A1

50

## BODY SHAPE DATA ACQUISITION SYSTEM

51
INPUT UNIT

54
MEMORY UNIT

56
MODIFICATION INSTRUCTION RECEPTION UNIT

52
MEASUREMENT UNIT

53
SELECTION UNIT

55
ACQUISITION UNIT

57
DISPLAY UNIT

Fig.3

| MEASURING NUMBER | GENDER | AGE | HEIGHT | WEIGHT | BMI | FAT PERCENTAGE | REFERENCE BODY SHAPE DATA |
|---|---|---|---|---|---|---|---|
| 1475 | 1 | 15 | 169.5 | 50.3 | 17.50867 | 9 | r0544.json |
| 1573 | 1 | 15 | 167.0 | 47.3 | 18.39546 | 6.9 | r5641.json |
| 1591 | 2 | 18 | 157.8 | 54.5 | 19.81208 | 10.3 | r8756.json |
| 1593 | 1 | 60 | 161.3 | 58.1 | 25.14632 | 12.9 | r0014.json |
| 1618 | 2 | 35 | 175.6 | 78.2 | 21.62141 | 7.5 | r2112.json |
| ⋮ | ⋮ | ⋮ | ⋮ | ⋮ | ⋮ | ⋮ | ⋮ |

Fig.4

Fig.5

Fig.6

THIRD
PRINCIPAL
COMPONENT

SECOND
PRINCIPAL
COMPONENT

FIRST
PRINCIPAL
COMPONENT

Fig.7

Fig.8

571

M

T

| HEIGHT | ///////// | 150 |
|---|---|---|
| CHEST | //////// | 97 |
| NECK CIRCUMFERENCE | /////// | 37 |
| NECK LENGTH | ///// | 12 |
| SHOULDER WIDTH | ///////// | 13 |
| SHOULDER SLOPE | ///// | 0.9 |
| ABDOMEN SHAPE | /////// | 1 |
| CHEST CIRCUMFERENCE | /////// | 86 |
| WAIST | ////// | 77 |
| ARM LENGTH | ///// | 26 |
| UPPER ARM CIRCUMFERENCE | ////////// | 15 |
| WRIST | ///// | 16 |
| BUTTOCK CIRCUMFERENCE | //////// | 112 |
| BUTTOCK HEIGHT | ///// | 36 |
| THIGH CIRCUMFERENCE | //// | 42 |
| LOWER LEG LENGTH | ////////// | 38 |
| CALF | ////// | 55 |

Fig.9

```
                    ┌──────────────────┐
                    │      START       │
                    └──────────────────┘
                             │
                             ▼
              ┌────────────────────────────┐
      S101 ───│    ACQUIRES BIOMETRIC       │
              │       INFORMATION          │
              └────────────────────────────┘
                             │
                             ▼
              ┌────────────────────────────┐
      S102 ───│   SELECT ONE REFERENCE     │
              │     BODY SHAPE DATA        │
              └────────────────────────────┘
                             │
                             ▼
              ╱──────────────────────────────╲   NO
      S103 ──│ CONTENTS OF THE MODIFICATION IN THE ├───┐
              ╲        PAST ARE STORED?        ╱       │
                            │ YES                      │
                            ▼                          │
              ┌────────────────────────────┐           │
              │  MODIFY THE REFERENCE BODY SHAPE │     │
      S104 ───│  DATA ACCORDING TO THE CONTENTS OF │   │
              │   THE MODIFICATION IN THE PAST   │     │
              └────────────────────────────┘           │
                            │ ◄───────────────────────┘
                            ▼
              ╱──────────────────────────────╲   NO
      S105 ──│     RECEIVE THE MODIFICAITON   ├───┐
              ╲  INSTRUCTIONS FROM THE USER?  ╱   │
                            │ YES                  │
                            ▼                      │
              ┌────────────────────────────┐       │
              │  MODIFY THE REFERENCE BODY SHAPE │ │
      S106 ───│  DATA ACCORDING TO THE RECEIVED │ │
              │    MODIFICATION INSTRUCTIONS    │ │
              └────────────────────────────┘       │
                            │                       │
                            ▼                       │
              ┌────────────────────────────┐         │
      S107 ───│    STORE THE RECEIVED       │        │
              │  MODIFICATION INSTRUCTIONS  │        │
              └────────────────────────────┘         │
                            │ ◄─────────────────────┘
                            ▼
              ┌────────────────────────────┐
      S108 ───│   ACQUIRE THE BODY SHAPE    │
              │     DATA OF THE USER        │
              └────────────────────────────┘
                            │
                            ▼
              ┌────────────────────────────┐
      S109 ───│   DISPLAY THE HUMAN         │
              │      BODY MODEL             │
              └────────────────────────────┘
                            │
                            ▼
                    ┌──────────────────┐
                    │     FINISH       │
                    └──────────────────┘
```

Fig.10

START

S111 — ACQUIRE BIOMETRIC INFORMATION

S112 — SELECT A PLURALITY OF REFERENCE BODY SHAPE DATA

S113 — SYNTHESIZE THE PLURALITY OF REFERENCE BODI SHAPE DATA

S114 — CONTENTS OF THE MODIFICATION IN THE PAST ARE STORED? → NO

YES

S115 — MODIFY THE COMPOSITE BODY SHAPE DATA ACCORDING TO THE CONTENTS OF THE MODIFICATION IN THE PAST

S116 — RECEIVE THE MODIFICAITON INSTRUCTIONS FROM THE USER? → NO

YES

S117 — MODIFY THE COMPOSITE BODY SHAPE DATA ACCORDING TO THE RECEIVED MODIFICATION INSTRUCTIONS

S118 — STORE THE RECEIVED MODIFICATION INSTRUCTIONS

S119 — ACQUIRE THE BODY SHAPE DATA OF THE USER

S120 — DISPLAY THE HUMAN BODY MODEL

FINISH

Fig.11

## INTERNATIONAL SEARCH REPORT

| | International application No. |
|---|---|
| | PCT/JP2020/005109 |

**A. CLASSIFICATION OF SUBJECT MATTER**
A61B 5/05(2006.01)i; A61B 5/107(2006.01)i
FI: A61B5/107 100; A61B5/05 B; A61B5/107 300

According to International Patent Classification (IPC) or to both national classification and IPC

**B. FIELDS SEARCHED**

Minimum documentation searched (classification system followed by classification symbols)
A61B5/04-5/05, A61B5/06-5/22

Documentation searched other than minimum documentation to the extent that such documents are included in the fields searched
| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2009-039289 A (TANITA CORP.) 26.02.2009 (2009-02-26) paragraphs [0001]-[0056], fig. 1-4 | 1-14 |
| A | JP 2005-110962 A (TANITA CORP.) 28.04.2005 (2005-04-28) paragraphs [0001]-[0046], fig. 1-7 | 1-14 |
| A | JP 2014-018444 A (TANITA CORP.) 03.02.2014 (2014-02-03) paragraphs [0001]-[0079], fig. 1-13 | 1-14 |
| A | WO 2017/168525 A1 (3D BODY LAB CO., LTD.) 05.10.2017 (2017-10-05) paragraphs [0001]-[0185], fig. 1-25 | 1-14 |

☐ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 April 2020 (02.04.2020) | 14 April 2020 (14.04.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

INTERNATIONAL SEARCH REPORT

Information on patent family members

International application No.

PCT/JP2020/005109

| Patent Documents referred in the Report | Publication Date | Patent Family | Publication Date |
|---|---|---|---|
| JP 2009-039289 A | 26 Feb. 2009 | (Family: none) | |
| JP 2005-110962 A | 28 Apr. 2005 | US 2005/0080352 A1 paragraphs [0001]- [0089], fig. 1-7 EP 1522259 A1 | |
| JP 2014-018444 A | 03 Feb. 2014 | US 2014/0025346 A1 paragraphs [0001]- [0167], fig. 1-13 EP 2687158 A1 CN 103565441 A | |
| WO 2017/168525 A1 | 05 Oct. 2017 | (Family: none) | |

Form PCT/ISA/210 (patent family annex) (January 2015)

28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019022352 A

- JP 5990820 B